# NOUVEAU FASCICULE DE BREVET EUROPEEN

(11) **EP 1 188 432 B2**
(45) Date de publication et mention de la décision concernant l'opposition: **22.06.2011**
(45) Mention de la délivrance du brevet: 14.02.2007
(21) Numéro de dépôt: 01402298.2
(22) Date de dépôt: 05.09.2001
(51) Int. Cl.: A61Q 5/04

(54) **Procédé de déformation permanente des matières kératinique mettant en oeuvre un agent absorbant organique**
Verfahren zum Dauerwellen keratischer Materialien unter Anwendung eines organischen Absorptionsmittels
Permanent wave process for keratinic materials using organic absorbing agent

(30) Priorité: 18.09.2000 FR 0011890
(43) Date de publication de la demande: 20.03.2002
(73) Titulaire: L'Oréal, 75008 Paris (FR)
(72) Inventeur: Nguyen, Ly-Lan, 94240 l'Hay les Roses (FR); Sabbagh, Anne, 92500 Rueil-Malmaison (FR); Devin-Baudoin, Priscille, 92170 Vanves (FR)
(74) Mandataire: Casalonga, Axel

(56) Documents cités:
- EP-A- 0 968 703
- WO-A-99/18922
- DE-A- 2 822 125
- JP-A- 05 017 322
- US-A- 5 531 987
- DATABASE WPI Derwent Publications Ltd., London, GB; AN 1993-071002 XP002173548 & JP 05 017322 A (T. OGA ET AL.), 26 janvier 1993 (1993-01-26)
- WENNINGER J.A. ET AL: 'International Cosmetic Ingredient Dictionary and Handbook', vol. 1, 1997, THE COSMETIC, TOILETRY, AND FRAGRANCE ASSOCIATION, WASHINGTON, DC article 'Cellulose Acetate, Ethylcellulose, Acrylates/Acrylamide Copolymer', pages 215,507 - &25
- WENNINGER J.A. ET AL: 'International Cosmetic Ingredient Dictionary and Handbook', vol. 2, 1997, THE COSMETIC, TOILETRY, AND FRAGRANCE ASSOCIATION, WASHINGTON, DC article 'Starch Diethylaminoethyl Ether, Polyacrylic Acid', pages 1339 & - 1065

## Description

L'invention a pour objet un nouveau procédé de traitement des matières kératiniques, en particulier des cheveux, pour obtenir une déformation permanente de ces dernières. Elle vise également un kit comprenant dans des compartiments séparés les différentes compositions nécessaires à ce type de traitement.

En matière de déformation permanente des cheveux, il est connu de réaliser, en un premier temps, sur les cheveux préalablement mis sous tension (à l'aide de rouleaux, bigoudis ou autres), l'ouverture des liaisons disulfure de la cystine de la kératine à l'aide d'une composition contenant un agent réducteur puis, généralement après un rinçage de la chevelure, de reconstituer lesdites liaisons disulfure, en appliquant une composition, généralement oxydante, permettant de fixer la coiffure. Cette technique permet ainsi de réaliser indifféremment soit une ondulation, soit un défrisage, soit un crêpage des cheveux.

Les compositions réductrices pour la déformation permanente des cheveux contiennent dans la plupart des cas, des actifs sensibilisants et irritants pour la peau. Ces compositions sont souvent liquides. Inévitablement, lorsqu'un tel produit est appliqué sur des cheveux enroulés sur bigoudis et autres, il coule sur le cuir chevelu favorisant les irritations cutanées.

Afin de remédier à ce problème, il a été tenté d'épaissir la composition réductrice. Néanmoins, pour que les actifs agissent de la racine à la pointe, il faut appliquer le produit épaissi avant d'enrouler la chevelure sur les bigoudis. Le temps de pose est alors très variable d'une mèche à l'autre et les résultats ne sont pas satisfaisants.

Par ailleurs, les cheveux sont très souvent plus sensibilisés aux pointes qu'à la racine. Les actifs de la composition réductrice pénètrent donc plus facilement à la pointe du cheveu et la frisure est en conséquence plus serrée en pointe qu'en racine. Une solution pour remédier à ce problème consiste à appliquer sur les parties sensibilisées un fluide protecteur qui limite la pénétration des actifs. Cette solution n'est toutefois pas satisfaisante dans la mesure où il est difficile de localiser précisément l'application du fluide. Il existe en outre un risque de rendre le cheveu trop glissant et de rendre ainsi la mise sous tension difficile.

A titre d'agents réducteurs dans les compositions réductrices, on utilise très généralement des thiols. Ces réducteurs thiolés ont une odeur désagréable. Des parfums ont été incorporés aux lotions réductrices afin de couvrir de façon relativement efficace cette odeur. Il est néanmoins indispensable de poser un bonnet sur la chevelure pendant le temps de pose afin de limiter la diffusion des odeurs. Lorsque ce bonnet est ôté, il se dégage de la chevelure une odeur concentrée de thiol et surtout de cheveu réduit qu'il est difficile de masquer.

Ainsi, il s'avère nécessaire d'améliorer les techniques de déformation permanente des cheveux notamment en remédiant aux problèmes ci-dessus.

Plus précisément, il existe un besoin pour un nouveau procédé permettant de réaliser une déformation permanente des cheveux exempte des inconvénients exprimés ci-dessus, et qui, en particulier, procure aux cheveux des déformations, des boucles ou des frisures régulières en limitant de manière acceptable les risques de sensibilisation et d'irritation cutanées ainsi que les odeurs désagréables dégagées lors de la pose.

De manière surprenante et inattendue, la demanderesse a découvert qu'il était possible de résoudre les problèmes décrits plus haut en utilisant notamment dans un procédé de déformation permanente des cheveux, une composition réductrice prête à l'emploi contenant un agent organique à propriété d'absorption définie.

L'invention a donc pour objet un nouveau procédé de traitement des matières kératiniques et notamment de déformation permanente des cheveux, comprenant, dans un premier temps, l'application d'une composition réductrice prête à l'emploi comprenant au moins un agent absorbant organique, puis après un temps de pose nécessaire à la réduction des matières kératiniques, l'application d'une composition fixatrice généralement oxydante.

Un autre objet de l'invention concerne un kit comprenant dans un premier compartiment un liquide réducteur comprenant au moins un agent réducteur, dans un second compartiment une composition fixatrice et, dans un troisième compartiment, un agent absorbant organique.

Selon la présente invention, on propose un nouveau procédé de traitement convenant à la déformation et/ou la mise en forme, et ceci de manière permanente, des matières kératiniques, et en particulier des cheveux, caractérisé en ce qu'il comprend les étapes suivantes :
(i) on mélange au moment de l'emploi, un liquide réducteur contenant au moins un agent réducteur avec un agent absorbant organique choisi parmi les carbométhylcelluloses de sodium réticules et les amidons modifiés pour former une composition réductrice (a); la concentration en agent absorbant organique dans la composition réductrice (a) étant comprise entre 7 et 11% en poids par rapport au poids total de la composition;
(ii) on applique dans un premier temps sur la matière kératinique à traiter la composition réductrice (a) associant au moins un agent réducteur et au moins ladit agent absorbant organique;
(iii) après un temps de repos nécessaire à la réduction de la matière kératinique, on applique une composition fixatrice (b);
(iv) après un temps de repos nécessaire à la fixation, on rince la matière kératinique ainsi traitée.

La matière kératinique peut être mise sous tension, notamment à l'aide de rouleaux, de bigoudis ou analogues, avant, pendant ou après l'application de la composition réductrice (a) de l'étape (ii).

Par agent absorbant, on entend tout composé susceptible de piéger rapidement une grande quantité d'eau. L'agent absorbant organique est donc généralement un composé hydrophile ou amphiphile.

Par agent absorbant au sens de la présente invention telle que revendiquée, on entend tout composé ayant une capacité statique d'absorption d'eau à température ambiante (25°C) supérieure ou égale à trois fois son poids.

De préférence, on choisit ces agents absorbants parmi les composés ayant une capacité statique d'absorption d'eau supérieure ou égale à cinq fois leur poids et préférentiellement supérieure ou égale à dix fois leur poids.

Le test pour mesurer ladite capacité statique d'absorption d'eau consiste, à température ambiante, à disposer régulièrement au fond d'un bécher de 150 ml, d'un diamètre d'environ 6 cm, le composé à tester en une quantité de 1 gramme ; à ajouter de l'eau sur la poudre en une quantité de 3 grammes ; et à laisser reposer, sans agiter, pendant 1 minute le mélange. S'il ne reste plus d'eau libre, c'est-à-dire d'eau surnageante, après ladite minute, le composé peut être considéré comme agent absorbant au sens de l'invention.

Le procédé de l'invention convient particulièrement bien à l'obtention d'une chevelure permanentée.

Appliqué à une chevelure saine, et même répété plusieurs fois, le procédé de l'invention présente pour avantages principaux, entre autres, de conduire, et ceci avec un moindre dégagement d'odeurs désagréables d'une part et d'une façon non irritante pour la peau et pour le cuir chevelu d'autre part, à de belles boucles ou frisures régulières.

D'autres caractéristiques, aspects et avantages de l'invention apparaîtront encore plus clairement à la lecture de la description détaillée qui va suivre, ainsi que de l'exemple concret, non limitatif, destiné à l'illustrer.

Bien que l'exposé qui suit s'articule essentiellement autour du cas particulier du traitement du cheveu, le procédé de l'invention est applicable à toute matière kératinique en générale, notamment cils, moustaches, poils, laine et autres.

La première étape du procédé de l'invention consiste en l'application d'une composition réductrice (a), avant, pendant ou après la mise sous tension des cheveux (au moyen de bigoudis, rouleaux ou autres).

La composition réductrice (a) prête à l'emploi est obtenue par un mélange extemporané, au moment de l'emploi, d'un liquide réducteur contenant au moins un agent réducteur et d'au moins un agent absorbant organique.

En particulier, on peut choisir l'agent absorbant parmi, seuls ou en mélange :
- les carboxyméthylcelluloses de sodium réticulés.

De tels produits sont notamment vendus par AVEBE sous la dénomination PRIMELLOSE.
- les amidons modifiés.

Les amidons naturels ne possèdent généralement pas une bonne capacité statique d'absorption d'eau ; il est généralement nécessaire de les modifier de manière à obtenir un agent absorbant au sens de l'invention. Une telle modification peut consister en un greffage de sels de sodium faiblement réticulés et/ou en une prégélatinisation.

Parmi les amidons modifiés susceptibles d'être utilisés, on peut citer les fécules de pommes de terre prégélatinisées quaternisées, les amidons de maïs prégélatinisés, les carboxyméthylamidons de pomme de terre réticulés, les phosphates de diamidon de manioc prégélatinisés et éventuellemen hydroxypropylés, les phosphates de diamidon de pomme de terre prégélatinisés et éventuellement acétylés.

Parmi les produits commercialement disponibles, on peut citer les produits vendus par AVEBE sous les dénominations PREGEL ou PRIMOGEL, ou ceux vendus par NATHIONAL STARCH sous la dénomination STRUCTURE ZEA.

Les agents absorbants particulièrement préférés sont notamment choisis parmi les carboxyméthylamidons.

Les carboxyméthylamidons peuvent se présenter notamment sous forme de poudre ou sous forme d'une suspension dans une huile hydrophobe. Par huile hydrophobe, on entend par exemple des huiles cosmétiquement acceptables, les huiles minérales, les esters d'acides gras, les huiles végétales, les huiles animales, les huiles de synthèse.

La composition réductrice (a) comprend entre 0,5 et 20%, de préférence 4 à 15% en poids d'agent réducteur par rapport au poids total de la composition.

De préférence, la composition réductrice (a) comprend au moins un agent réducteur thiolé choisi parmi l'acide thioglycolique, l'acide thiolactique ou leurs sels, la cystéine, la cystéamine et le thioglycolate de glycérol.

Le pH de la composition réductrice (a) est compris entre 5 et 11, de préférence entre 6 et 10, et encore plus préférentiellement entre 8,5 et 9,5.

La composition réductrice (a) comprend en outre avantageusement au moins un agent alcalin, pouvant notamment être choisi parmi l'ammoniaque, la monoéthanolamine, la diéthanolamine, la triéthanolamine, l'isopropanolamine, la propanediamine-1,3, un carbonate ou bicarbonate d'un métal alcalin ou alcalinoterreux ou d'ammonium, un carbonate organique tel que le carbonate de guanidine ou encore un hydroxyde d'un métal alcalin ou alcalinoterreux, utilisé seul ou en mélange.

Selon un aspect particulièrement avantageux du procédé selon l'invention, on applique la composition réductrice (a) sur une chevelure humide. Dans la pratique, les cheveux sont éventuellement réhumidifiés avant application de ladite composition réductrice.

Lorsqu'on opère à partir d'une composition réductrice préparée au moment de l'emploi, celle-ci épaissit au contact du cheveu humide, car l'agent absorbant organique se gonfle progressivement d'eau. La composition épaissit suffisamment rapidement pour ne pas couler sur le cuir chevelu. Pendant ce gonflement, l'actif réducteur pénètre progressivement dans le cheveu, moins rapidement qu'un réducteur liquide, mais sur la totalité du cheveu de la racine jusqu'à la pointe. Le mélange épaissi déposé sur les cheveux ressemble à un glaçage. Ce « glaçage » permet de réduire les effluves des thiols et de cheveux réduits. Ceci permet de laisser agir la composition réductrice sans poser de bonnet sur la chevelure pendant le temps de pose.

On laisse au repos la chevelure traitée avec la composition réductrice (a) pendant un temps suffisant à la réduction des cheveux. Ce temps est généralement de l'ordre de 10 à 15 minutes.

Selon un autre aspect particulièrement avantageux du procédé de l'invention, préalablement à l'application de la composition fixatrice (b) de l'étape (ii) mais après un certain temps de repos des cheveux traitée avec la composition réductrice (a), la chevelure est rincée soigneusement, généralement à l'eau, jusqu'à élimination complète de la composition réductrice.

Une autre étape importante du procédé de l'invention consiste en l'application d'une composition fixatrice (b). Cette composition fixatrice (b) peut contenir tout agent oxydant connu en soi.

Avantageusement, la composition fixatrice (b) comprend au moins un agent oxydant choisi parmi le peroxyde d'hydrogène, le peroxyde d'urée, les bromates de métaux alcalins, les persels tels que les perborates et les persulfates.

La composition fixatrice (b) contient également des agents régulateurs de pH, de manière à maintenir un pH acide.

Les compositions fixatrice (b) ou réductrice (a) peuvent contenir, en outre, de préférence, des additifs choisis parmi les polymères anioniques, non ioniques, amphotères, les tensioactifs, les silicones, des cires, des épaississants, des agents de pénétration, des alcools gras, des dérivés de lanoline, des céramides, des ingrédients actifs, des agents anti-chute, anti-pelliculaires, des agents de mise en suspension, des agents séquestrants, des agents opacifiants, des agents stabilisants, des colorants, des filtres solaires siliconés ou non, des conservateurs, des parfums.

Dans le but notamment d'améliorer les propriétés cosmétiques des cheveux, la composition fixatrice (b) ou la composition réductrice (a) peuvent en outre contenir des polymères cationiques. Par polymères cationiques, on entend tout polymère contenant des groupements cationiques ou des groupements ionisables en groupements cationiques.

A titre de polymères cationiques utilisables dans le cadre de l'invention, on peut citer plus particulièrement les polymères du type polyamine, polyamidoamide, polyammonium quaternaire, polymères cationiques siliconés, polyalkylèneimine, en particulier les polyéthylèneimines, les polymères contenant des motifs vinylpyridine ou vinylpyrridinium, les condensats de polyamine et d'épichlorhydrine, les polyuréylène quaternaires, les cyclopolymères et les dérivés de la chitine.

Parmi tous les polymères cationiques susceptibles d'être utilisés dans le cadre de la présente invention, on préfère mettre en oeuvre les cyclopolymères, en particulier les homopolymères du chlorure de diméthyldiallylammonium, commercialisés sous la dénomination MERQUAT® 100 par la Société MERCK, et certains polymères de diammonium quaternaire comme l'HEXADIMETRINECHLORIDE de la Société CHIMEX.

Selon l'invention, on peut également utiliser des polymères cationiques sous forme de latex ou de pseudolatex, c'est-à-dire sous forme d'une dispersion de particules de polymères insolubles.

Selon l'invention, le ou les polymères cationiques peuvent représenter de 0,01% à 20% en poids, de préférence de 0,1 à 15% en poids du poids total de la composition finale.

Dans la dernière étape du procédé selon l'invention (étape (iii)), on rince la chevelure ainsi traitée après un temps de repos nécessaire à la fixation de la chevelure. Ce temps est généralement de l'ordre de 5 minutes.

On enlève de la chevelure les moyens mécaniques (rouleaux, bigoudis et analogues) qui maintenaient sous tension dans la forme désirée les cheveux tout au long du traitement, avant ou après rinçage de la composition fixatrice, la suppression des moyens de mise sous tension pouvant éventuellement être suivie de la réapplication d'une certaine quantité de composition fixatrice (b).

On peut obtenir finalement une chevelure présentant par exemple de belles boucles régulières permanentes.

Le procédé se termine par un séchage naturel ou par tout autre moyen de séchage (infrarouge, sèche-cheveux et autres), afin d'obtenir une belle frisure.

L'invention pourra être mieux comprise à l'aide de l'exemple qui suit et qui constitue un mode de mise en oeuvre avantageux du procédé conforme à l'invention.

### Exemple

On réalise les compositions suivantes :

Pour la composition réductrice, on prépare d'une part la partie liquide réducteur A, et d'autre part la partie B.

**Composition réductrice**

| *Partie liquide A :* | | |
|---|---|---|
| Ammoniaque (à 20,5% en ammoniaque) | | 12,1 g |
| Acide thioglycolique | | 9,4 g |
| Alcool oléique oxyéthyléné (20 moles d'oxyde d'éthylène) | | 1,1 g |
| Parfum | | 0,5 g |
| Acide diéthylène triamine pentacétique, sel pentasodique en solution aqueuse à 40% | | 0,4 g |
| Eau déminéralisée | qs | 90 g |
| | | |

| *Partie poudre B:* | | |
|---|---|---|
| Primojel | | 10 g |
| (fournisseur AVEBE, carboxyméthylamidon de pomme de terre, sel de sodium faiblement réticulé) | | |

**Composition fixatrice**

| | | |
|---|---|---|
| Peroxyde d'hydrogène en solution à 50% | | 4,8 g |
| Oxyde de lauryl diméthylamine en solution aqueuse (30%) | | 2,15 g |
| P-éthoxy acétanilide | | 0,05 g |
| Parfum | | 0,2 g |
| Acide citrique | | 0,1 g |
| Sulfate de 8-hydroxyquinoléine | | 0,0125 g |
| Eau déminéralisée | qs | 100 g |

Au moment de l'emploi, on mélange le liquide réducteur A et la poudre B en agitant vigoureusement. On laisse le mélange reposer 1 minute.

La suspension fluide obtenue est à nouveau agitée avant l'application sur les cheveux humides.

La composition réductrice épaissit au contact du cheveu humide, préalablement enroulé sur bigoudis.

La composition est laissée 15 minutes sur les cheveux.

La chevelure est ensuite rincée.

La composition fixatrice est ensuite appliquée sur tous les bigoudis. On respecte un temps de pose de 5 minutes avant de rincer la chevelure.

La frisure obtenue présente des boucles régulières. Lors de l'application, des émanations d'odeur désagréable sont considérablement diminuées tout comme l'irritation du cuir chevelu.

## Revendications

1. Procédé de traitement convenant à la déformation et/ou la mise en forme, et ceci de manière permanente, des matières kératiniques, et en particulier des cheveux, **caractérisé en ce qu'**il comprend les étapes suivantes :
(i) on mélange au moment de l'emploi, un liquide réducteur contenant au moins un agent réducteur avec un agent absorbant organique choisi parmi carboxyméthylcelluloses de sodium réticulés et les amidons modifiés pour former une composition réductrice (a); la concentration en agent absorbant organique dans la composition réductrice (a) est comprise entre 7 et 11% en poids par rapport au poids total de la composition.
(ii) on applique dans un premier temps sur la matière kératinique à traiter la composition, réductrice (a) associant au moins un agent réducteur et au moins ledit agent absorbant organique, les moyens nécessaire à la mise sous tension mécanique de la matière kératinique étant mis en oeuvre avant, pendant ou après ladite application ;
(iii) après un temps de repos nécessaire à la réduction de la matière kératinique, on applique une composition fixatrice (b) ;
(iv) après un temps de repos nécessaire à la fixation, on rince la matière kératinique ainsi traitée.

2. Procédé selon la revendication précédente, **caractérisé en ce que** l'agent absorbant organique est un carboxyméthylamidon.

3. Procédé selon la revendication 1, **caractérisé en ce que** la composition prête à l'emploi réductrice (a) est laissée à reposer pendant au moins 1 minute après le mélange.

4. Procédé selon la revendication 1, **caractérisé en ce que** l'agent absorbant organique est utilisé en suspension dans une huile hydrophobe.

5. Procédé selon l'une quelconque des revendications précédentes, **caractérisé en ce que** la composition (a) est appliquée sur une chevelure humide.

6. Procédé selon l'une quelconque des revendications précédentes, **caractérisé en ce que** préalablement à l'application de la composition fixatrice (b), la chevelure est rincée.

7. Procédé selon l'une quelconque des revendications précédentes, **caractérisé par le fait que** la composition réductrice (a) comprend au moins un agent réducteur choisi parmi l'acide thioglycolique, l'acide thiolactique, la cystéine, la cystéamine et le thioglycolate de glycérol.

8. Procédé selon l'une quelconque des revendications précédentes, **caractérisé par le fait que** la composition fixatrice (b) comprend au moins un agent oxydant choisi parmi le peroxyde d'hydrogène, le peroxyde d'urée, les bromates de métaux alcalins, les persels tels que les perborates et persulfates.

9. Procédé selon l'une quelconque des revendications précédentes,**caractérisé par le fait que** la composition réductrice (a) comprend en outre au moins un agent alcalin, pouvant notamment être choisi parmi l'ammoniaque, la monoéthanolamine, la diéthanolamine, la triéthanolamine, l'isopropanolamine, la propanediamine-1,3, un carbonate ou bicarbonate de métal alcalin ou alcalinoterreux ou d'ammonium, un carbonate organique tel que le carbonate de guanidine ou encore un hydroxyde d'un métal alcalin ou alcalinoterreux, utilisé seul ou en mélange.

10. Procédé selon l'une quelconque des revendications précédentes,**caractérisé par le fait que** la composition réductrice (a) ou fixatrice (b) contient, en outre, des additifs choisis parmi les tensioactifs, les polymères cationiques, anioniques, non ioniques, amphotères, les silicones, des cires, des épaississants, des agents de pénétration, des alcools gras, des dérivés de lanoline, des céramides, des ingrédients actifs, des agents anti-chute, anti-pelliculaires, des agents de mise en suspension, des agents séquestrants, des agents opacifiants, des agents stabilisants, des colorants, des filtres solaires siliconés ou non, des conservateurs, des parfums.

11. Kit pour le traitement convenant à la déformation et/ou la mise en forme, et ceci de manière permanente, des matières kératiniques et en particulier des cheveux, comprenant, dans un premier compartiment, un liquide réducteur comprenant au moins un agent réducteur, dans un second compartiment, une composition fixatrice (b) telle que définie précédemment et, dans un troisième compartiment, au moins un agent absorbant organique choisi parmi les carboxyméthylcelluloses de sodium réticulés, les amidons modifiés la concentration en agent absorbant organique dans la composition réductrice (a) est comprise entre 7 et 11% en poids par rapport au poids total de la composition.

## Claims

1. Treatment process suitable for the permanent deformation and/or shaping of keratinous substances and in particular the hair, **characterized in that** it comprises the following stages:
(i) at the time of use, a reducing liquid comprising at least one reducing agent is mixed with an organic absorbing agent chosen from crosslinked sodium carboxymethylcelluloses and modified starches to form a reducing composition (a); the concentration of organic absorbing agent in the reducing composition (a) is between 7 and 11%, by weight with respect to the total weight of the composition
(ii) in a first step, the reducing composition (a), combining at least one reducing agent and at least the said organic absorbing agent, is applied to the keratinous substance to be treated, the means necessary for placing the keratinous substance under mechanical tension being employed before, during or after the said application;
(iii) after a leave-in time necessary for the reduction of the keratinous substance, a neutralizing composition (b) is applied;
(iv) after a leave-in time necessary for the neutralization, the keratinous substance thus treated is rinsed.

2. Process according to the preceding claim, **characterized in that** the organic absorbing agent is a carboxymethylstarch.

3. Process according to Claim 1, **characterized in that** the ready-for-use reducing composition (a) is left to stand for at least 1 minute after the mixing.

4. Process according to Claim 1, **characterized in that** the organic absorbing agent is used in suspension in a hydrophobic oil.

5. Process according to any one of the preceding claims, **characterized in that** the composition (a) is applied to wet hair.

6. Process according to any one of the preceding claims, **characterized in that**, prior to the application of the neutralizing composition (b), the hair is rinsed.

7. Process according to any one of the preceding claims, **characterized in that** the reducing composition (a) comprises at least one reducing agent chosen from thioglycolic acid, thiolactic acid, cysteine, cysteamine and glycerol thioglycolate.

8. Process according to any one of the preceding claims, **characterized in that** the neutralizing composition (b) comprises at least one oxidizing agent chosen from hydrogen peroxide, urea hydrogen peroxide, alkali metal bromates, or persalts, such as perborates and persulphates.

9. Process according to any one of the preceding claims, **characterized in that** the reducing composition (a) additionally comprises at least one alkaline agent which can be chosen in particular from ammonia, monoethanolamine, diethanolamine, triethanolamine, isopropanolamine, 1,3-propanediamine, an alkali metal or alkaline earth metal or ammonium carbonate or bicarbonate, an organic carbonate, such as guanidine carbonate, or an alkali metal or alkaline earth metal hydroxide, used alone or as a mixture.

10. Process according to any one of the preceding claims, **characterized in that** the reducing composition (a) or the neutralizing composition (b) additionally comprises additives chosen from surfactants, cationic, anionic, nonionic or amphoteric polymers, silicones, waxes, thickeners, penetrating agents, fatty alcohols, lanolin derivatives, ceramides, active ingredients, agents for combating hair loss, antidandruff agents, suspending agents, sequestering agents, opacifying agents, stabilizing agents, colourants, silicone or non-silicone sunscreen agents, preservatives or fragrances.

11. Kit for the treatment suitable for the permanent deformation and/or shaping of keratinous substances and in particular the hair comprising, in a first compartment, a reducing liquid comprising at least one reducing agent, in a second compartment, a neutralizing composition (b) as defined above and, in a third compartment, at least one organic absorbing agent chosen from crosslinked sodium carboxymethylcelluloses, modified starches; the concentration of organic absorbing agent in the reducing composition (a) is between 7 and 11%, by weight with respect to the total weight of the composition.

## Patentansprüche

1. Verfahren zur Behandlung, das für die Verformung und/oder Formgebung von Keratinsubstanzen und insbesondere Haaren auf permanente Weise geeignet ist, **dadurch gekennzeichnet, dass** es die folgenden Schritte umfasst:
(i) bei der Anwendung wird eine reduzierende Flüssigkeit, die mindestens ein Reduktionsmittel enthält, mit einem organischen Absorptionsmittel vermischt, das unter vernetzten Natriumcarboxymethylcellulosen und modifizierten Stärken ausgewählt ist, um eine reduzierende Zusammensetzung (a) zu bilden, wobei die Konzentration des organischen Absorptionsmittels in der reduzierenden Zusammensetzung (a) im Bereich von 7 bis 11 Gew.-%, bezogen auf das Gesamtgewicht der Zusammensetzung, liegt.
(ii) die reduzierende Zusammensetzung (a), die mindestens ein Reduktionsmittel in Kombination mit zumindest dem organische Absorptionsmittel enthält, wird in einem ersten Schritt auf die zu behandelnde Keratinsubstanz aufgetragen, wobei vor, während oder nach dem Aufbringen die Mittel, die erforderlich sind, um die Keratinsubstanz unter mechanischer Spannung zu halten, verwendet werden;
(iii) nach einer für die Reduktion der Keratinsubstanz erforderlichen Einwirkzeit wird eine fixierende Zusammensetzung (b) aufgebracht;
(iv) nach einer für die Fixierung erforderlichen Einwirkzeit wird die so behandelte Keratinsubstanz gespült.

2. Verfahren nach dem vorhergehenden Anspruch, **dadurch gekennzeichnet, dass** es sich bei dem organischen Absorptionsmittel um eine Carboxymethylstärke handelt.

3. Verfahren nach Anspruch 1, **dadurch gekennzeichnet, dass** die gebrauchsfertige reduzierende Zusammensetzung (a) nach dem Mischen mindestens 1 Minute ruhen gelassen wird.

4. Verfahren nach Anspruch 1, **dadurch gekennzeichnet, dass** das organische Absorptionsmittel in Suspension in einem hydrophoben Öl verwendet wird.

5. Verfahren nach einem der vorhergehenden Ansprüche, **dadurch gekennzeichnet, dass** die Zusammensetzung (a) auf die feuchten Haare aufgebracht wird.

6. Verfahren nach einem der vorhergehenden Ansprüche, **dadurch gekennzeichnet, dass** vor dem Aufbringen der fixierenden Zusammensetzung (b) das Haar gespült wird.

7. Verfahren nach einem der vorhergehenden Ansprüche, **dadurch gekennzeichnet, dass** die reduzierende Zusammensetzung (a) mindestens ein Reduktionsmittel enthält, das unter Thioglycolsäure, Thiomilchsäure, Cystein, Cysteamin und Glycerinthioglycolat ausgewählt ist.

8. Verfahren nach einem der vorhergehenden Ansprüche, **dadurch gekennzeichnet, dass** die fixierende Zusammensetzung (b) mindestens ein Oxidationsmittel enthält, das unter Wasserstoffperoxid, Harnstoffperoxid, Alkalimetallbromaten, Salzen von Persäuren, wie Perboraten und Persulfaten, ausgewählt ist.

9. Verfahren nach einem der vorhergehenden Ansprüche, **dadurch gekennzeichnet, dass** die reduzierende Zusammensetzung (a) ferner mindestens ein Alkalisierungsmittel enthält, das insbesondere unter Ammoniak, Monoethanolamin, Diethanolamin, Triethanolamin, Isopropanolamin, 1,3-Diaminopropan, Alkalioder Erdalkalicarbonaten, Alkali- oder Erdalkalibicarbonaten, Ammoniumcarbonat, Ammoniumbicarbonat, organischen Carbonaten, wie Guanidincarbonat, oder Alkali- oder Erdalkalihydroxiden ausgewählt ist, die einzeln oder im Gemisch verwendet werden.

10. Verfahren nach einem der vorhergehenden Ansprüche, **dadurch gekennzeichnet, dass** die reduzierende Zusammensetzung (a) oder die fixierende Zusammensetzung (b) ferner Zusatzstoffe enthalten, die unter den grenzflächenaktiven Stoffen, kationischen, anionischen, nichtionischen, amphoteren Polymeren, Siliconen, Wachsen, Verdickungsmitteln, Penetrationsmitteln, Fettalkoholen, Lanolinderivaten, Ceramiden, wirksame Bestandteile, Wirkstoffen gegen Haarausfall, Antischuppenmitteln, Suspendiermitteln, Maskierungsmitteln, Trübungsmitteln, Stabilisatoren, Farbmitteln, siliconierten oder nicht siliconierten Sonnenschutzfiltern, Konservierungsmitteln oder Parfums ausgewählt sind.

11. Kit für die Behandlung, das für die Verformung und/oder Formgebung von Keratinsubstanzen und insbesondere Haaren auf dauerhafte Weise geeignet ist und in einer ersten Abteilung eine reduzierende Flüssigkeit enthält, die mindestens ein Reduktionsmittel aufweist, in einer zweiten Abteilung eine fixierende Zusammensetzung (b), wie sie oben definiert wurde, und in einer dritten Abteilung mindestens ein organisches Absorptionsmittel, das unter den vernetzten Natriumcarboxymethylcellulosen, modifizierten Stärken ausgewählt ist, wobei die Konzentration des organischen Absorptionsmittels in der reduzierenden Zusammensetzung (a) im Bereich von 7 bis 11 Gew.-%, bezogen auf das Gesamtgewicht der Zusammensetzung, liegt.
